# EUROPÄISCHE PATENTSCHRIFT

(11) **EP 3 630 003 B1**
(45) Veröffentlichungstag und Bekanntmachung des Hinweises auf die Patenterteilung: **27.11.2024**
(21) Anmeldenummer: 18716478.5
(22) Anmeldetag: 12.03.2018
(51) Int. Cl.: A61C 19/045

(54) **KIEFERGELENKAUFZEICHNUNGSSYSTEM**
MANDIBULAR JOINT RECORDING SYSTEM
SYSTÈME D'ENREGISTREMENT DE L'ARTICULATION TEMPORO-MANDIBULAIRE

(30) Priorität: 24.05.2017 AT 2202017
(43) Veröffentlichungstag der Anmeldung: 08.04.2020
(73) Patentinhaber: Gamma Medizinisch-Wissenschaftliche Fortbildungs-GmbH, 3400 Klosterneuburg (AT)
(72) Erfinder: SLAVICEK, Christian, 3400 Klosterneuburg (AT)
(74) Vertreter: Fox, Tobias
(86) Internationale Anmeldenummer: PCT/AT2018/000013
(87) Internationale Veröffentlichungsnummer: WO 2018/213857

(56) Entgegenhaltungen:
- DE-A1- 102004 002 953
- DE-U1- 202004 004 955
- JP-A- 2001 112 743
- US-A- 5 143 086
- US-A1- 2013 157 218

## Beschreibung

Die Erfindung betrifft ein Kiefergelenkaufzeichnungssystem (Kondylograph) zur gelenknahen elektronischen Registrierung der Beweglichkeit eines Unterkiefers eines Patienten, wobei durch den Schädel des Patienten ein kartesisches Koordinatensystem, ausgehend von einem Nullpunkt als Halbierungspunkt einer Strecke zwischen den beiden Kiefergelenken auf der Gelenkachse sowie aus einer in der durch die Gelenkachse und einen vorderen Referenzpunkt definierten Horizontalebene liegenden und nach frontal zeigenden x-Achse, einer y-Achse entlang der Verbindungslinie der beiden Kiefergelenke und einer z-Achse entlang der Hochachse des Schädels, definiert wird, wobei der Kondylograph einen oberen Bogen, welcher fest mit dem Schädel des Patienten verbindbar ist und welcher mindestens eine Kamera an mindestens einer Patientenseite aufweist, sowie einen unteren Bogen umfasst, welcher fest mit dem Unterkiefer verbindbar ist und mindestens einen Referenzmarker im Sichtbereich der mindestens einen Kamera aufweist, wobei der oder die Referenzmarker jeweils an einem linken und/oder an einem rechten Ende des unteren Bogens angeordnet ist bzw. sind, und wobei das linke und das rechte Ende des unteren Bogens fest miteinander verbunden sind.

Bei der Aufzeichnung der Unterkieferbewegung ist besonders die Bewegung der gemeinsamen Scharnierachse beider Kiefergelenke von Bedeutung. Das menschliche Kiefergelenk kann sowohl rotatorische als auch translatorische Bewegungen darstellen. Funktionelle Bewegungsmuster des Unterkiefers bestehen daher auch zumeist immer aus einer Kombination dieser beiden Komponenten. Aus diagnostischer und therapeutischer Sicht der Zahnmedizin ist im besonderen die Translationskomponente der Bewegung von Bedeutung. Translationsbewegungen dienen vorwiegend als Berechnungsbasis für patientenindividuelle Einstellungen von dentalen Bewegungssimulatoren im Artikulator. Die Rotationskomponente in der Bewegung bewirkt in der graphischen Schreibung eine Verzerrung der Aufzeichnungsergebnisse, die Reproduzierbarkeit vermindert sich und die diagnostischen Ergebnisse werden zweifelhaft. Daher ist es vorteilhaft, Bewegungen des Unterkiefers direkt auf der Rotationsachse des Unterkiefers zu schreiben.

Die bisher bekannten Vorrichtungen zur Registrierung der Unterkieferbewegungen in bezug auf den Schädel eines Patienten dienen der exakten Erfassung der Gelenkbewegung des Unterkiefers für diagnostische Zwecke und der Ermittlung von Kenndaten für dentalmedizinische Artikulatoren. Zusätzlich sollen auch Aussagen über den pathologischen Zustand der Unterkiefergelenke getroffen werden können. Dadurch können abnorme Bewegungsabläufe der Kiefergelenke durch den Einfluss von Muskulatur oder der Knorpelscheibe erkannt werden, um daraus eine Diagnose zu stellen und eine Therapieplanung abzuleiten. Geräte zum Zweck der Registrierung der Unterkieferbewegung werden in sogenannte "gelenknahe" und "gelenkferne" Aufzeichnungssysteme unterschieden. Gelenknahe Systeme messen direkt auf der patientenindividuell lokalisierten Gelenkachse in einem Messbereich von 3 mm oder auf einer Gelenkachse, die angenähert mittels anatomischer Schädelpunkte gefunden wurde. Gelenkferne Aufzeichnungssysteme messen nicht in Gelenknähe, sondern beispielsweise im Vorfeld des Gesichts. Die Lokalisation der Scharnierachse erfolgt zumeist virtuell, d.h. vom Computer berechnet. Die Bewegungsbahnen der Gelenkachse werden bei solchen Systemen mathematisch auf die Scharnierachse rückgerechnet.

Seit den 1980er und 1990er Jahren haben Experimente zum Filmen der Kiefergelenkbewegung mit Hilfe von Lichtquellen stattgefunden. Aus der DE 10 2004 015 383 A1 ist ein Gerät bzw. eine gelenkferne Aufzeichnungsmethodik bekannt, bei welchem bzw. bei welcher der Patient einen mit seinem Kopf verbundenen Rahmen trägt, der Sende- und Empfangseinrichtungen irgendeiner Art zur Aussendung und zum Empfang elektronischer, optischer oder akustischer Signale aufweist, und wobei mit dem Unterkiefer des Patienten ein weiteres Gestell verbunden wird, welches ebenfalls Sende- und Empfangseinrichtungen aufweist. Beide Gestelle sind mit einem Auswertecomputer verbunden. Nachteil dieser Lehre ist, dass die Gestelle aufwendig zu montieren sind, relativ schwer und frontlastig ausgeführt sein müssen, da beide über Kabel mit einer Auswerteeinrichtung verbunden sind, und dass ferner die rudimentären elektronischen Sendemittel eine Begrenzung der Messgenauigkeit darstellen. Die Messgenauigkeit gelenkferner Aufzeichnungssysteme wird auch durch die systemnotwendige geometrisch/mathematische Umrechnungen von der Position der Messsensorik auf die Scharnierachse der Kiefergelenke vermindert. Ein weiterer Nachteil dieser gelenkfernen Registriermethodik ist die Berechnung der individuellen Scharnierachse und die in Konsequenz verwendete virtuelle Scharnierachse und die sich daraus ergebenden Ungenauigkeiten bzw. systematischen Fehler, da eine direkte mechanische Überprüfung der Scharnierachslokalisation mit solchen Systemen nur mit erheblichem Zusatzaufwand möglich ist. Weiters ist es in der Lehre und Ausbildung von didaktischer Bedeutung, Studentinnen mittels direkter Einstellbarkeit die Methodik der Scharnierachslokalisation zu demonstrieren.

Die EP 1 981 403 B1 zeigt ein gelenknahes registrierendes Gerät, bei welchem der Kopf des Patienten temporär fest mit einem stationären Gerät verbunden wird, welches den Patienten über Kameras von oben und von seinen beiden Seiten aus filmt, während der Patient ein temporär fest mit dem Unterkiefer verbundenes Gestell trägt, welches in alle Raumrichtungen etwa vierzehn Grafikmarkierungen aufweist. Nachteilig ist hier, dass ein Labor oder eine Zahnarztpraxis, welches bzw. welche die Unterkiefergelenkmessung durchführen möchte, einen vergleichsweise großräumigen Platz für das stationäre Gerät freihalten muss.

Die DE 10 2004 002 953 A1 zeigt einen Kondylographen, bei welchem am Schädel befestigte Ultraschallsensoren die Signale von Ultraschallsendern an einem Bogen am Kiefer aufzeichnen.

DE 20 2004 004 955 U1 lehrt einen Kondylographen, bei welchem am Schädel befestigte Empfangsmittel das Signal von einem zugeordneten Sendemittel, das am Kiefer befestigt ist, aufzeichnen. Die US 2013/0157218 A1 offenbart etwas dazu ähnliches, wobei die Empfangsmittel Kameras sind.

Die US 5,143,086 A zeigt eine Vorrichtung, wobei am Schädel befestigte Kameras die Bewegung des Gebisses eines Patienten filmen.

Die Erfindung zielt darauf ab, einen Kondylographen wie eingangs angeführt zu schaffen, welcher die Nachteile des Standes der Technik überwindet. Weiterhin soll die Vorrichtung wenig störanfällig sein, eine hohe Messgenauigkeit bieten, leicht zu bedienen und ergonomisch für den Patienten sein. Gleichzeitig sollen die Herstellungs- und Wartungskosten des Kondylographen so gering wie möglich gehalten werden.

Der erfindungsgemäße Kondylograph erreicht dies dadurch, dass an mindestens einer Patientenseite zwei Kameras am oberen Bogen vorgesehen sind, welche Sichtachsen aufweisen, die einen in einer parallel zur x-z-Ebene liegenden Ebene liegenden Winkel α zwischen etwa 30 und etwa 150 Grad zueinander einschließen, und wobei die Kameras jeweils auf den mindestens einen Referenzmarker ihrer Patientenseite gerichtet sind und der oder die Referenzmarker auf eine nach Gesichtslandmarken anatomisch ermittelte Gelenkachse oder auf die individuelle Scharnierachse des Kiefergelenks des Patienten und einen Bereich von bis zu etwa 3 mm herum einstellbar ist bzw. sind.

Die Erfindung wird durch den angefügten unabhängigen Anspruch 1 definiert. Vorteilhafte Ausführungsformen ergeben sich aus den angefügten abhängigen Ansprüchen.

Eine bevorzugte Ausführungsform des Kondylographen zeichnet sich dadurch aus, dass der oder die Referenzmarker Kugeln ist bzw. sind.

In einer Ausführungsform der Erfindung sind die Referenzmarker eine oder mehrere Kugeln auf einer oder beiden Patientenseiten, wobei die Kugeln einer Patientenseite in vordefinierter Distanz beabstandet sind.

Zur weiteren Ausgestaltung der Erfindung liegt der Winkel α der Kameras zwischen etwa 80 und 100 Grad.

Bevorzugt ist in einer Ausgestaltung der Erfindung, dass die Kameras Infrarotkameras und die Referenzmarker mit einer Infrarotfarbe beschichtet sind, und wobei der Kondylograph eine oder mehrere Lichtquellen aufweist, durch welche der oder die Referenzmarker mit Licht im infraroten Spektralbereich beleuchtbar ist bzw. sind. Alternativ kann der oder die Referenzmarker aktiv lichtemittierend ausgeführt werden, beispielsweise in Form von innenliegenden Infrarot-LEDs.

Eine bevorzugte Ausführungsform des Kondylographen zeichnet sich dadurch aus, dass die Kameras pro Patientenseite in einem in allen Raumrichtungen im wesentlichen geschlossenen Gehäuse angeordnet sind.

In einer Ausführungsform der Erfindung ist das oder sind die Gehäuse schwenkbar am oberen Bogen befestigt.

Zur weiteren Ausgestaltung der Erfindung ist der Kondylograph Teil einer kondylographischen Vorrichtung, wobei der obere Bogen mittels eines Kabels, bevorzugt nach dem USB-System, mit einer computerartigen Steuerungs- und Aufzeichnungseinrichtung verbunden ist.

Weiterhin ist es bei einer bevorzugten Ausführungsform der Erfindung möglich, dass die Datenübermittlung von Kameras und die Steuersignale an die Kameras und die Lichtquellen von und zu einer computerartigen Steuerungs- und Aufzeichnungseinrichtung über eine Funkverbindung erfolgt.

Bevorzugt ist in einer Ausgestaltung der Erfindung, dass die kondylographische Vorrichtung ein Anzeigegerät aufweist.

In einer Ausführungsform der Erfindung weist die Steuerungs- und Aufzeichnungseinrichtung eine Software auf, mit welcher Kenndaten für dentalmedizinische Artikulatoren automatisch ermittelbar sind.

Die Erfindung wird nachstehend anhand eines in den Zeichnungen dargestellten Ausführungsbeispieles näher erläutert. Es zeigen: Fig. 1 eine schematische Seitenansicht eines Schädels eines Patienten mit teilweise eingezeichnetem Koordinatensystem, Fig. 2 eine perspektivische Ansicht eines Artikulator-Unterteils mit eingezeichnetem Koordinatensystem, Fig. 3 und Fig. 4 Vorder- und Seitenansicht des erfindungsgemäßen Kondylographen, Fig. 5 bis Fig. 8 Außenansichten des Gehäuses, welches zwei Kameras beinhaltet, und Fig. 9 eine schematische Ansicht eines Referenzmarker-Paares.

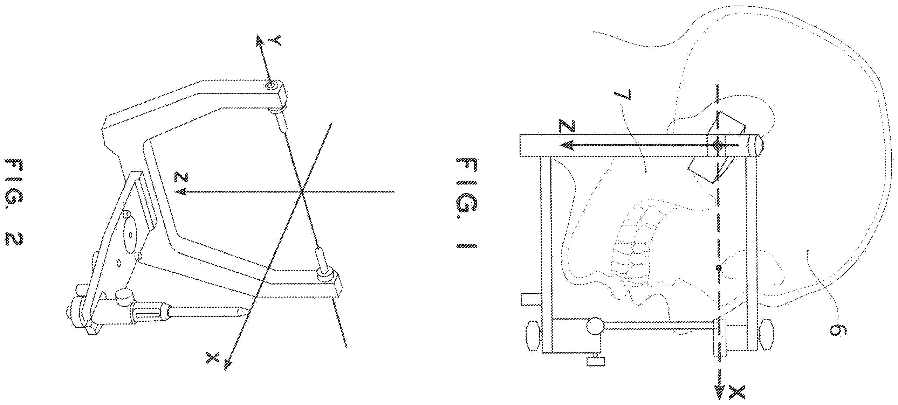

Der Schädel zusammen mit dem Unterkiefer in zentrierter Gelenkposition des Patienten definiert ein kartesisches Koordinatensystem, an welchem sich die Bestandteile des erfindungsgemäßen Kondylographen orientieren. Die gemeinsame Scharnierachse des rechten und des linken Kiefergelenkes bilden die y-Achse dieses Koordinatensystems. Es ist in Fig. 1 durch die gewählte Perspektive nicht zu sehen bzw. weist die y-Achse senkrecht in die Papierebene hinein. Die x-Achse liegt senkrecht zur y-Achse und verläuft entlang der Verbindungslinie zwischen Hinterkopf und Nase bzw. von der Gelenkachse auf Höhe eines vorderen Referenzpunktes des Patientenschädels nach frontal. Die Gelenkachse ist üblicherweise die gemeinsame Scharnierachse beider Kiefergelenke in zentrischer Kiefergelenkposition; der vordere Referenzpunkt ist üblicherweise der Orbitalpunkt als der tiefste Punkt des Vorderrandes der linken Augenhöhle. Wiederum senkrecht zur x- und zur y-Achse liegt die z-Achse, welche in der Hochachse des Schädels 6 des Patienten liegt bzw. im wesentlichen parallel zur längsten Erstreckung der Wirbelsäule eines Patienten im stehenden oder geradesitzenden Zustand mit erhobenem Kopf. In Fig. 2 ist dasselbe Koordinatensystem am technischen Modell eines Unterkiefers 7 gezeigt. Alle Achsen stehen senkrecht aufeinander, was zur Erläuterung oder Ausübung der Erfindung jedoch nicht wesentlich ist. Selbst im modellhaften Idealfall bewegen sich die Bestandteile des Unterkiefers 7 eines Patienten sowohl in der x-z-Ebene als auch in y-Richtung. Kieferbewegungen in y-Richtung sind also grundsätzlich nicht auszuschließen und sollen von der erfindungsgemäßen Vorrichtung mit ausreichender Genauigkeit ebenso gemessen werden können.

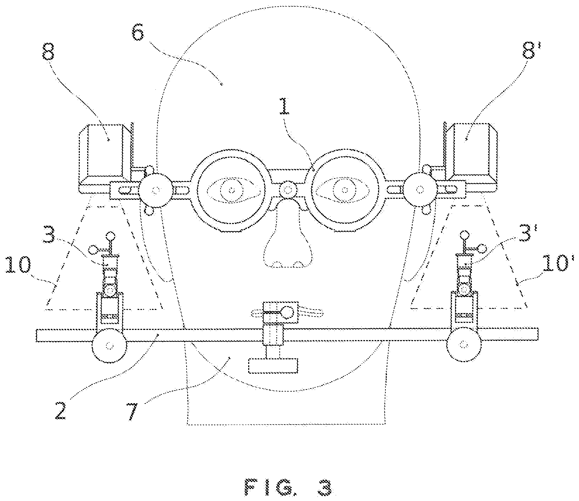

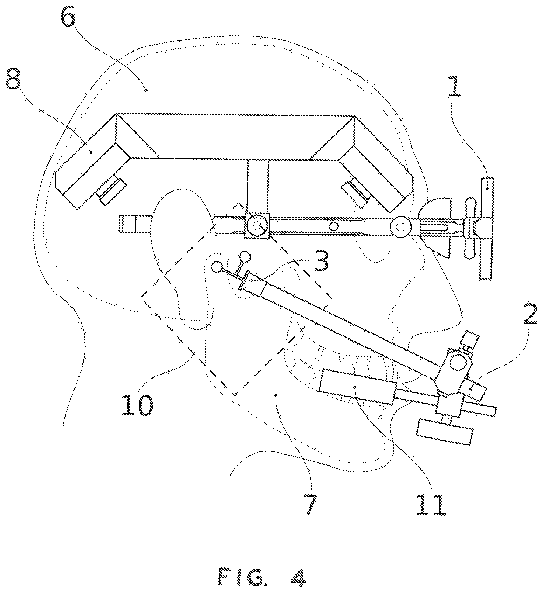

Gemäß Fig. 3 und Fig. 4 umfasst der Kondylograph zwei Teile, und zwar einen unteren Bogen 2, der zum Beispiel über eine Zahnklemme 11 temporär fest mit dem Unterkiefer 7 verbunden ist, und einen oberen Bogen 1, welcher temporär fest mit dem Schädel 6 des Patienten verbunden werden kann. Der obere Bogen 1 lässt sich in der gezeigten Ausführungsvariante wie eine Brille ruhend auf der Nasenwurzel und den Ohren befestigen. Zusätzliche Befestigungsmittel sind denkbar. Der obere Bogen 1 kann auch wie eine Brille mit je einem Rahmenteil vor jeweils einem Auge des Patienten gestaltet sein, wie in den Fig. 3 und 4 gezeigt, um dem Patienten auch während der Messung einen normalen ungehinderten Blick auf die Umgebung zu ermöglichen, er kann aber auch von jeder anderen beliebigen Form sein, solange sich der obere Bogen 1 auch auf die beiden Patientenseiten links und rechts erstreckt. An den Teilen des oberen Bogens 1, die sich über eine Patientenseite erstrecken, ist ein Gehäuse 8 befestigt, in welchem sich zwei Kameras 13 befinden. Im an dem oberen Bogen 1 befestigten Zustand blicken die beiden Kameras 13 aus dem Gehäuse 8 heraus auf Teile des unteren Bogens 2. Sowohl der obere Bogen 1 als auch der untere Bogen 2 überdecken einen Winkelbereich von 180 Grad in der x-y-Ebene um das Gesicht des Patienten herum. Im befestigten Zustand liegen die beiden Bogen 1, 2 in etwa parallel zueinander.

Alternativ kann die Kamera bzw. können die Kameras 13 oder ein Gehäuse 8 mit innenliegenden Kameras 13 am unteren Bogen 2 angebracht sein.

Zur einfachen Handhabung ist der untere Bogen 2 an im wesentlichen nur einem Punkt an den unteren Zähnen bzw. dem Gaumen des Patienten und somit mit seinem Unterkiefer 7 durch Klebung, Klemmung oder andere temporäre Fixierung fest verbunden. Auch der untere Bogen 2 kann eine beliebige Form annehmen, vorteilhaft ist jedoch, dass er aus Gründen der Gewichtsersparnis und somit der leichten Handhabung und Tragbarkeit möglichst leicht und daher aus leichtem, relativ dünn ausgebildetem Material hergestellt ist. Auch der untere Bogen 2 führt um den Schädel 6 des Patienten herum, so dass sich zumindest die Enden 3, 3' des unteren Bogens 2 an den beiden Seiten in Höhe der Kiefergelenke des Patienten befinden. Der untere Bogen 2 ist vorteilhafterweise einstückig ausgebildet, was die Herstellung vereinfacht und für eine dauerhafte Verwendung sorgt; er kann jedoch auch aus wenigen Teilen zusammengesetzt sein, solange alle zusammengefügten Teile eine einzige feste Konstruktion bilden. An den Enden 3, 3' des unteren Bogens 2, die sich an den Seiten des Patienten befinden und etwa auf der anatomisch lokalisierten Gelenkachse oder der individuellen Scharnierachse des Kiefergelenks des Patienten, die mithin auf der Höhe des Punktes der Gelenkachse des Patienten und einen Bereich von bis zu maximal 3 mm darum liegen, sind an jeder Seite mindestens ein Referenzmarker 4 angeordnet, welcher sich jeweils in den sich kreuzenden Sichtfeldern der Kameras 13 des Gehäuses 8 befindet. Für eine bessere Messung ist ein maximaler Abstand weniger als 1 mm zur patientenindividuellen Scharnierachse wünschenswert. Durch einfache mechanische Stellmittel lassen sich die Enden 3, 3' bzw. die Referenzmarker 4 auf den gewünschten Bereich bzw. Position schnell und individuell kurz vor der Messung einstellen.

Bei der alternativen Ausführungsform (nicht gezeigt) sind die Referenzmarker 4 am oberen Bogen 1 angebracht. Die Kameras 13 am unteren Bogen 2 werden in besonders leichter Bauweise dort angeordnet, wo die Referenzmarker 4 bei der ersten Ausführungsform sind, und zwar an den Enden 3, 3' des unteren Bogens 2, die etwa auf der anatomisch lokalisierten Gelenkachse oder der individuellen Scharnierachse des Kiefergelenks des Patienten liegen, also auf der Höhe des Punktes der Gelenkachse des Patienten und einen Bereich von bis zu maximal 3 mm darum. Der genaue Punkt wird zweckmäßig nicht durch eine der Kameras 13, sondern durch den Mittelpunkt der Verbindungslinie zwischen z.B. zwei Kameras 13 auf jeder Patientenseite berührt. Auch bei dieser zweiten Ausführungsform befinden sich die obenliegenden Referenzmarker 4 letztlich in den sich kreuzenden Sichtfeldern der Kameras 13.

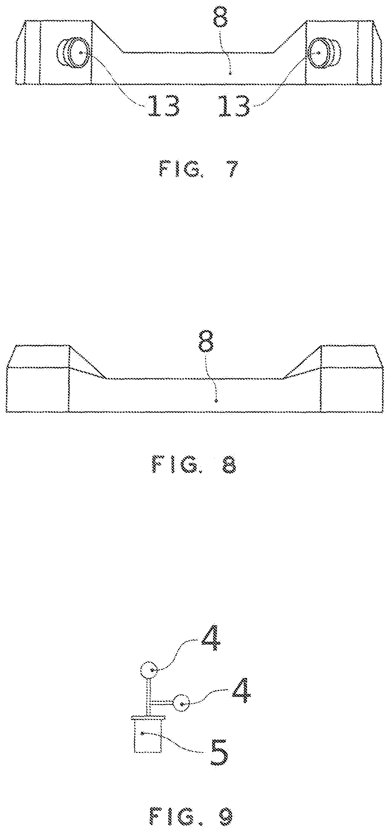

Der von beiden Kameras 13 sichtbare Bereich 10 erfasst somit die Bewegung eines oder auch mehrerer Referenzmarker 4 (siehe Fig. 9), während der Patient vorgeschriebene Bewegungen des Kiefers ausführt. Die Erfindung löst die Aufgabe mit einem Referenzmarker 4, der ein geometrischer Punkt sein kann und keine Musterung zu sein braucht. Die Verwendung von zwei oder mehr Referenzmarker 4 pro Patientenseite kann jedoch von Vorteil sein (siehe unten); die Messung einer Rotationskomponente bei der Kieferbewegung wird erst durch mindestens zwei Referenzmarker 4 pro Seite möglich. Eine entsprechende Ausführungsform ist in den Fig. 3 und 4 gezeigt, wobei die beiden Referenzmarker 4 pro Patientenseite nicht nur in x-Richtung, sondern auch in y- und z-Richtung voneinander beabstandet sind.

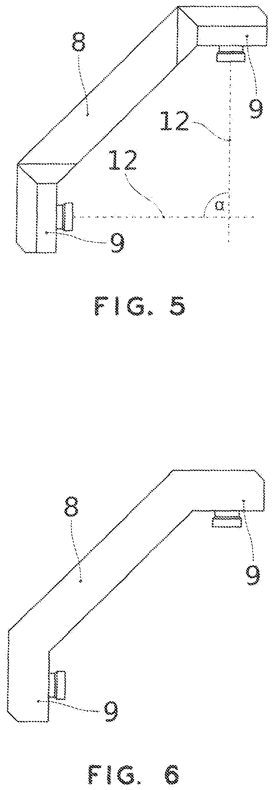

Gemäß Fig. 5, einer Seitenansicht des Gehäuses 8, befinden sich die Kameras 13 in Boxen 9 an den Enden des Gehäuses 8, die bereits einen Winkel zueinander vorgeben können. Dieses ist daher knochenförmig ausgebildet, wobei andere Formen auch denkbar sind. Die Sichtachsen 12 der Kameras 13 schneiden sich unter einem Winkel α, der im gezeigten Beispiel 90 Grad beträgt. Andere Winkelbereiche sind ebenso möglich. So kann der Winkel auch einen wählbaren Wert, zum Beispiel zwischen etwa 30 und 150 Grad haben, wodurch jede Bewegung des gefilmten Referenzmarkers in der x-z-Ebene von den Kameras 13 erfasst wird.

Fig. 6 zeigt die Rückansicht des Gehäuses 8 mit den beiden Boxen 9, Fig. 7 zeigt die Untersicht des Gehäuses 8 mit jeweils einer Öffnung für die Kameras 13. Fig. 8 zeigt das Gehäuse 8 in Draufsicht, welches ein in allen Raumrichtungen im wesentlichen geschlossenes Gehäuse 8 darstellt, so dass ausreichend Schutz für die Kameras 13 sowie eine vordefinierte feststehende Beabstandung und Winkelstellung der beiden Kameras 13 bewirkt wird, ferner durch zumindest relative Wasserdichtheit eine gute Abwaschbarkeit oder Wischdesinfektion dieses Bauteiles des Kondylographen und schließlich eine verminderte Gefahr, sich bei der Handhabung des erfindungsgemäßen Kondylographen an seinen Bestandteilen zu verletzen.

Auch der obere Bogen 1 sollte möglichst leicht ausgeführt sein, um die Tragbarkeit für den Patienten zu verbessern. Das heißt, es sollten möglichst viele Bestandteile aus leichten Materialien gefertigt sein und diese Materialien sparsam verwendet werden. Die elektronischen Bestandteile bzw. die Bestandteile, die an der Messung tatsächlich teilnehmen, sollten möglichst einfach und miniaturisiert ausgeführt sein.

Gemäß Fig. 9 bestehen die Referenzmarker 4 aus Kugeln, welche über einen Adapter 5 mit dem unteren Bogen 2 verbunden sind. Es hat sich als vorteilhaft herausgestellt, dass pro Patientenseite zwei Kugeln verwendet werden, die mindestens, wie in Fig. 9 gezeigt, im mit dem unteren Bogen 2 verbundenen Zustand in x-Richtung und dazu gegebenenfalls in negativer y-Richtung voneinander beabstandet sind, um eine dreidimensionale Bewegung des Unterkiefers 7 und auch eine Verdrehung des Unterkiefers 7 während seiner Bewegung erfassbar zu machen. Im Idealfall ist nur eine Beabstandung in x-Richtung erforderlich; um ein gegenseitiges Verdecken der Kugeln in Praxis jedoch mit Sicherheit auszuschließen, sind sie in allen drei Raumrichtungen voneinander entfernt. Vorteil der Erfindung ist, dass die Referenzmarker 4 keine weiteren optischen Merkmale aufweisen. So sind beispielsweise Musterungen oder andere besondere Formgebungen oder eine höhere Anzahl von Referenzmarker 4 nicht erforderlich.

Es hat sich herausgestellt, dass für eine schnelle und präzise Auswertung der Messergebnisse eine automatische Bildauswertung des Materials der Kameras 13 vorgenommen wird. Wiederum hat sich dabei herausgestellt, dass die Kameras 13 die Referenzmarker 4 dann für die Auswertesoftware gut sichtbar filmen, wenn im infraroten Spektralbereich gearbeitet wird. Das heißt, das Gehäuse 8 ist entweder zusätzlich zu den Kameras 13 mit Lichtquellen (nicht gezeigt) ausgestattet, welche den Sichtbereich 10 der Kameras 13 ausreichend mit infrarotem Licht ausleuchten, oder die Referenzmarker 4 sind aktiv ausgeführt und emittieren eigenständig Licht. LED-Lichtquellen haben den Vorteil, die Frequenz des ausgestrahlten Lichtes genau zu definieren, leicht und kostengünstig zu sein. Die Referenzkugeln sind mit einer Infrarotfarbe beschichtet, und die Kameras 13 sind im infraroten Spektralbereich besonders sensitiv.

Alternativ sind die Referenzmarker 4 selbst mit innenliegenden Infrarot-Lichtquellen, z.B. Infrarot-LEDs, ausgestattet. Dies ist vorteilhaft, da einerseits die Temperaturentwicklung um die Kameras 13 drastisch abnimmt sowie der Sichtbereich auf die exakt benötigte Frequenz eingestellt werden kann; so können auch Hintergrundinformationen über Thresholding besser herausgefiltert werden. Es wird auch weniger Energie benötigt und das Gewicht am oberen Bogen reduziert.

Durch das Arbeiten im Infrarot-Bereich kann die Messung bei nahezu beliebigem Umgebungslicht stattfinden. Dies hat den Vorteil, dass die vorhandenen Licht- und Schattenverhältnisse in der Zahnarztpraxis in keiner Weise für die Messung selbst angepasst oder verändert werden müssen. So ist die Handhabung des erfindungsgemäßen Kondylographen erleichtert. Um diesen Vorteil abermals zu steigern, werden die Enden des unteren Bogens 3, 3' oder der gesamte untere Bogen 2 mit einer Farbe beschichtet, die infrarotes Licht besonders stark absorbiert. Die Referenzkugeln treten dadurch deutlicher im Sichtfeld der Kameras 13 hervor.

In wenigen und einfachen Schritten ist somit eine konkrete Messung durchzuführen. Der Patient kann stehend oder bequem sitzend zum einen den oberen Bogen 1 aufsetzen und erhält zum anderen den unteren Bogen 2 an seinem Unterkiefer 7 befestigt. In den meisten Fällen sind die Kameras 13 und die Referenzmarker 4 bereits in einer richtigen räumlichen Anordnung zueinander. Gegebenenfalls wird mit einfachen mechanischen Mitteln nachjustiert. Der erfindungsgemäße Kondylograph erfordert kein stationäres Gerät, keine aufwendigen und bewegungshindernden oder bewegungsverändernden Verbindungen von Gerätschaften zum Patienten. Es sind auch keine störenden Verbindungen zwischen den beiden Bögen erforderlich. Lediglich ein oder zwei Kabel (eins pro Patientenseite) führen vom oberen Bogen 1 zum Computer oder einer Rechnereinheit, die als Bestandteil des Systems mitgeliefert wird (beides nicht gezeigt), in welchem bzw. in welcher die Auswertung der Messergebnisse automatisch erfolgt. Dieses Kabel kann dem USB-System folgen und ist daher ein vergleichsweise dünnes, leichtes und sehr flexibles Kabel, welches weder den Patienten stören noch seine Kieferbewegung verändern wird. Ferner ist ein USB-Kabel leicht austauschbar. Auch das geringe Gewicht des unteren Bogens 2 stört den Patienten nicht. Die auf dem Auswertecomputer installierte Software steuert nun die Kameras 13 im Gehäuse 8 an und zeichnet das Bildmaterial der Kameras 13 auf, während der Patient die vorgegebenen Kieferbewegungen ausführt. Keine weiteren Änderungen am Patienten oder an der Zahnarztpraxis sind erforderlich. Die Messung ist anschließend bereits abgeschlossen.

Es ist denkbar, die Datenübermittlung von Kameras 13 und die Steuersignale an die Kameras 13 und die Lichtquellen auch über eine Funkverbindung herzustellen, wobei eine miniaturisierte Elektronik zum Einsatz kommt, welche, inklusive Batterie, ebensowenig Gewicht zum Gesamtgewicht des Kondylographen beiträgt.

Es ist auch daran gedacht, das Gehäuse 8 schwenkbar auf dem oberen Bogen 1 zu befestigen. So kann das Gehäuse 8 und mit ihm der Sichtbereich 10 der Kameras 13 zum Beispiel in der x-z-Ebene geschwenkt werden, wenn sich ebenso die Referenzmarker 4 am unteren Bogen 2 auf einer anderen Höhe oder in einem anderen Bereich an der Patientenseite befinden und dort die Messung der Kieferbewegung vorteilhafter erscheint. Hierzu ist das Gehäuse 8 über ein Gelenk, welches vordefinierte Schwenkmittel des Gehäuses 8 zulässt und temporär arretiert, verbunden.

Es ist möglich, ein Gehäuse 8 nur rechts oder nur links am Patienten vorzusehen und für die Messung zu verwenden, da dadurch aufgrund der Statik eines menschlichen Kiefers bereits die gesamte Kieferbewegung erfassbar ist. Eine mögliche Dysfunktion an einer Patientenseite kann jedoch nicht durch eine Messung an der anderen Seite erkannt werden. Daher ist die Messung durch Kamerapaare jeweils an beiden Patientenseiten vorteilhaft. Dazu wird ein zweites Gehäuse 8 an der dann noch freien Seite des oberen Bogens 1 (sowie entsprechende Referenzmarker 4 am unteren Bogen 2) befestigt, um auch parallel oder abwechselnd auch an der nun zweiten Seite des Patienten zu messen.

Vorteilhafterweise ist der Kondylograph direkt oder über eine computerartige Steuerungs- und Aufzeichnungseinrichtung, welche die Steuerung und Auswertung vornimmt, mit einem Anzeigegerät verbunden, welches der betreuenden Person Messergebnisse, den Zustand des Kondylographen oder lediglich ein rudimentäres Signal, welches die erfolgreiche Messung bestätigt, anzeigt. Aus der gefilmten Bewegung der Referenzmarker 4 lassen sich mit einer Software ohne menschliches Zutun und ohne nennenswerten Zeitablauf Kenndaten für dentalmedizinische Artikulatoren automatisch ermitteln. Zudem können die Aufzeichnungsdaten bei beidseitiger und gleichzeitiger Registrierung auf einen beliebigen Interkondylarabstand (beispielsweise den tatsächlichen Interkondylarabstand des Patienten) umgerechnet werde. Diese Kenndaten können in Form eines Ausdruckes, einer Datei oder ähnlichem praktischerweise gespeichert und abgegeben werden.

## Patentansprüche

1. Kondylograph zur elektronischen gelenknahen Registrierung der Beweglichkeit eines Unterkiefers (7) eines Patienten, wobei durch den Schädel (6) des Patienten ein kartesisches Koordinatensystem, ausgehend von einem Nullpunkt als Halbierungspunkt einer Strecke zwischen den beiden Kiefergelenken auf der Gelenkachse und bestehend aus einer in der durch die Gelenkachse und einem dritten vorderen Referenzpunkt definierten Horizontalebene liegenden und nach frontal zeigenden x-Achse, einer y-Achse entlang der Verbindungslinie der beiden Kiefergelenke und einer z-Achse entlang der Hochachse des Schädels (6), definiert wird, wobei der Kondylograph einen oberen Bogen (1), welcher fest mit dem Schädel (6) des Patienten verbindbar ist und welcher mindestens eine Kamera (13) an mindestens einer Patientenseite aufweist, sowie einen unteren Bogen (2) umfasst, welcher fest mit dem Unterkiefer (7) verbindbar ist und mindestens einen Referenzmarker (4) im Sichtbereich (10) der mindestens einen Kamera (13) aufweist, wobei der oder die Referenzmarker (4) jeweils an einem linken und/oder an einem rechten Ende (3, 3') des unteren Bogens (2) angeordnet ist bzw. sind, und wobei das linke und das rechte Ende (3, 3') des unteren Bogens (2) fest miteinander verbunden sind, **dadurch gekennzeichnet, dass** an mindestens einer Patientenseite zwei Kameras (13) am oberen Bogen (1) vorgesehen sind, welche Sichtachsen (12) aufweisen, die einen in einer parallel zur x-z-Ebene liegenden Ebene liegenden Winkel α zwischen etwa 30 und etwa 150 Grad zueinander einschließen, und wobei die Kameras (13) jeweils auf den mindestens einen Referenzmarker (4) ihrer Patientenseite gerichtet sind und der oder die Referenzmarker (4) auf eine nach Gesichtslandmarken anatomisch ermittelte Gelenkachse oder auf die individuelle Scharnierachse des Kiefergelenks des Patienten und einen Bereich von bis zu etwa 3 mm herum einstellbar ist bzw. sind.

2. Kondylograph nach Anspruch 1, **dadurch gekennzeichnet, dass** der oder die Referenzmarker (4) Kugeln ist bzw. sind.

3. Kondylograph nach Anspruch 1 oder 2, **dadurch gekennzeichnet, dass** die Referenzmarker (4) ein oder mehrere Kugeln auf einer oder beiden Patientenseiten sind, wobei die Kugeln einer Patientenseite in vordefinierter Distanz beabstandet sind.

4. Kondylograph nach einem der Ansprüche 1 bis 3, **dadurch gekennzeichnet, dass** der Winkel α der Kameras (13) zwischen etwa 80 und etwa 100 Grad liegt.

5. Kondylograph nach einem der Ansprüche 1 bis 4, **dadurch gekennzeichnet, dass** die Kameras (13) Infrarotkameras und die Referenzmarker (4) mit einer Infrarotfarbe beschichtet sind, und wobei der Kondylograph Infrarotlichtquellen aufweist, durch welche der oder die Referenzmarker (4) mit Licht im infraroten Spektralbereich beleuchtbar ist bzw. sind.

6. Kondylograph nach einem der Ansprüche 1 bis 4, **dadurch gekennzeichnet, dass** der oder die Referenzmarker (4) lichtemittierend ausgeführt sind und innenliegend eine Infrarot-Lichtquelle aufweisen.

7. Kondylograph nach einem der Ansprüche 1 bis 6, **dadurch gekennzeichnet, dass** die Kameras (13) pro Patientenseite in einem in allen Raumrichtungen im wesentlichen geschlossenen Gehäuse (8) angeordnet sind.

8. Kondylograph nach Anspruch 7, **dadurch gekennzeichnet, dass** das oder die Gehäuse (8) schwenkbar am oberen Bogen (1) befestigt ist bzw. sind.

9. Kondylographische Vorrichtung, bestehend aus einem Kondylographen, nach einem der Ansprüche 1 bis 8, **dadurch gekennzeichnet, dass** der obere Bogen (1) mittels eines Kabels, bevorzugt nach dem USB-System, mit einer computerartigen Steuerungs- und Aufzeichnungseinrichtung verbunden ist.

10. Kondylographische Vorrichtung, bestehend aus einem Kondylographen, nach einem der Ansprüche 1 bis 8, **dadurch gekennzeichnet, dass** die Datenübermittlung von Kameras (13) und die Steuersignale an die Kameras (13) und die Lichtquellen von und zu einer computerartigen Steuerungs- und Aufzeichnungseinrichtung über eine Funkverbindung erfolgt.

11. Kondylographische Vorrichtung nach Anspruch 9 oder 10, **dadurch gekennzeichnet, dass** die Vorrichtung ein Anzeigegerät aufweist.

12. Kondylographische Vorrichtung nach einem der Ansprüche 9 bis 11, **dadurch gekennzeichnet, dass** die Steuerungs- und Aufzeichnungseinrichtung eine Software aufweist, mit welcher Kenndaten für dentalmedizinische Artikulatoren automatisch ermittelbar sind.

## Claims

1. Condylograph for the electronic recording, close to the joint, of the mobility of a patient's lower jaw (7), wherein a Cartesian coordinate system is defined by the patient's skull (6), starting from a zero point as the bisecting point of a line between the two jaw joints on the joint axis and consisting of an x-axis lying in the horizontal plane defined by the joint axis and a third reference point pointing in a frontal direction, a y-axis along the connecting line of the two jaw joints, and a z-axis along the vertical axis of the skull (6), wherein the condylograph comprises an upper bracket (1) which can be firmly connected to the patient's skull (6) and which has at least one camera (13) on at least one side of the patient, and a lower bracket (2) which can be firmly connected to the lower jaw (7) and which has at least one reference marker (4) in the field of view (10) of the at least one camera (13), wherein the reference marker or markers (4) are respectively arranged at a left and/or right end (3, 3') of the lower bracket (2), and wherein the left and right ends (3, 3') of the lower bracket (2) are firmly connected to one another,
**characterised in that**
on at least one side of the patient, two cameras (13) are provided on the upper bracket (1) which have viewing axes (12) which enclose an angle α between about 30 and about 150 degrees with one another in a plane lying parallel to the x-z plane, and wherein the cameras (13) are respectively directed towards the at least one reference marker (4) on their patient side, and the reference marker or markers (4) can be adjusted to a joint axis determined anatomically according to facial landmarks or to the individual hinge axis of the patient's jaw joint and a range of up to about 3 mm round it.

2. Condylograph according to claim 1, **characterised in that** the reference marker or markers (4) is/are spheres.

3. Condylograph according to claim 1 or 2, **characterised in that** the reference markers (4) are one or more spheres on one or both sides of the patient, wherein the spheres on one side of the patient are spaced apart at a predefined distance.

4. Condylograph according to one of the claims 1 to 3, **characterised in that** the angle α of the cameras (13) is between about 80 and about 100 degrees.

5. Condylograph according to one of the claims 1 to 4, **characterised in that** the cameras (13) are infrared cameras and the reference markers (4) are coated with an infrared colour, and wherein the condylograph has infrared light sources by which the reference marker or markers (4) can be illuminated with light in the infrared spectral range.

6. Condylograph according to one of the claims 1 to 4, **characterised in that** the reference marker or markers (4) are designed to be light-emitting and have an infrared light source inside.

7. Condylograph according to one of the claims 1 to 6, **characterised in that** the cameras (13) on each patient side are arranged in a housing (8) which is essentially closed in all spatial directions.

8. Condylograph according to claim 7, **characterised in** the housing or housings (8) are attached to the upper bracket (1) in a pivoted manner.

9. Condylographic device consisting of a condylograph according to one of the claims 1 to 8, **characterised in that** the upper bracket (1) is connected to a computer-like control and recording device by means of a cable, preferably according to the USB system.

10. Condylographic device consisting of a condylograph according to one of the claims 1 to 8, **characterised in that** the data transmission from cameras (13) and the control signals to the cameras (13) and the light sources from and to a computer-type control and recording device takes place via a radio connection.

11. Condylographic device according to claim 9 or 10, **characterised in that** the device has a display device.

12. Condylographic device according to one of the claims 9 to 11, **characterised in that** the control and recording device has software with which characteristic data for dental articulators can be determined automatically.

## Revendications

1. Axiographe destiné à l'enregistrement électronique, à proximité de l'articulation, de la mobilité d'une mandibule (7) d'un patient, un système de coordonnées cartésiennes, qui part d'un point zéro considéré comme le point de bissection d'une distance entre les deux articulations de mâchoire sur l'axe d'articulation et est constitué d'un axe x pointant vers l'avant et situé dans le plan horizontal défini par l'axe d'articulation et par un troisième point de référence antérieur, d'un axe y s'étendant le long de la ligne de liaison des deux articulations de mâchoire, et d'un axe z s'étendant le long de l'axe vertical du crâne (6), étant défini grâce au crâne (6) du patient, l'axiographe comprenant un arc supérieur (1) qui peut être relié de manière fixe au crâne (6) du patient et qui présente au moins une caméra (13) au niveau d'au moins un côté de patient, et un arc inférieur (2) qui peut être relié de manière fixe à la mandibule (7) et qui présente au moins un marqueur de référence (4) dans la plage de vision (10) de la au moins une caméra (13), le ou les marqueur(s) de référence (4) étant agencé(s) respectivement sur une extrémité gauche et/ou sur une extrémité droite (3, 3') de l'arc inférieur (2), et les extrémités gauche et droite (3, 3') de l'arc inférieur (2) étant reliées l'une à l'autre de manière fixe, **caractérisé en ce que**, au niveau d'au moins un côté de patient, deux caméras (13) présentant des axes de vision (12) qui forment l'un par rapport à l'autre un angle α situé dans un plan parallèle au plan x-z et compris entre environ 30 et environ 150 degrés sont fournies sur l'arc supérieur (1), et les caméras (13) étant dirigées respectivement vers le au moins un marqueur de référence (4) de leur côté de patient et le ou les marqueur(s) de référence (4) pouvant être ajusté(s) dans une plage pouvant aller jusqu'à environ 3 mm sur un axe d'articulation déterminé anatomiquement d'après des repères topologiques de visage ou sur l'axe de charnière individuel de l'articulation de mâchoire du patient.

2. Axiographe selon la revendication 1, **caractérisé en ce que** le ou les marqueur(s) de référence (4) est/sont des billes.

3. Axiographe selon la revendication 1 ou 2, **caractérisé en ce que** le(s) marqueur(s) de référence (4) est/sont une ou plusieurs bille(s) sur un côté ou les deux côtés du patient, les billes d'un côté du patient étant espacées selon une distance prédéfinie.

4. Axiographe selon l'une quelconque des revendications 1 à 3, **caractérisé en ce que** l'angle α des caméras (13) est compris entre environ 80 et environ 100 degrés.

5. Axiographe selon l'une quelconque des revendications 1 à 4, **caractérisé en ce que** les caméras (13) sont des caméras infrarouges et les marqueurs de référence (4) sont revêtus d'une peinture infrarouge, l'axiographe présentant des sources de lumière infrarouge grâce auxquelles le ou les marqueur(s) de référence (4) peu(ven)t être éclairé(s) avec de la lumière du domaine spectral infrarouge.

6. Axiographe selon l'une quelconque des revendications 1 à 4, **caractérisé en ce que** le ou les marqueur(s) de référence (4) est/sont conçu(s) pour émettre de la lumière et présente(nt) en son/leur sein une source de lumière infrarouge.

7. Axiographe selon l'une quelconque des revendications 1 à 6, **caractérisé en ce que** les caméras (13) sont agencées pour chaque côté de patient dans un boîtier (8) essentiellement fermé dans toutes les directions spatiales.

8. Axiographe selon la revendication 7, **caractérisé en ce que** le ou les boîtier(s) (8) est/sont fixé(s) de manière pivotante sur l'arc supérieur (1).

9. Dispositif axiographique constitué d'un axiographe selon l'une quelconque des revendications 1 à 8, **caractérisé en ce que** l'arc supérieur (1) est relié à un dispositif de commande et d'enregistrement de type ordinateur au moyen d'un câble utilisant de manière préférée le système USB.

10. Dispositif axiographique constitué d'un axiographe selon l'une quelconque des revendications 1 à 8, **caractérisé en ce que** la transmission de données à partir des caméras (13) et les signaux de commande adressés aux caméras (13) et aux sources de lumière passent par une liaison radio à partir de et vers un dispositif de commande et d'enregistrement de type ordinateur.

11. Dispositif axiographique selon la revendication 9 ou 10, **caractérisé en ce que** le dispositif présente un appareil d'affichage.

12. Dispositif axiographique selon l'une quelconque des revendications 9 à 11, **caractérisé en ce que** le dispositif de commande et d'enregistrement comprend un logiciel permettant de déterminer automatiquement des données caractéristiques propres aux articulateurs dentaires.
